# EUROPEAN PATENT APPLICATION

(11) **EP 4 005 564 A1**
(43) Date of publication of application: **01.06.2022**
(21) Application number: 20848233.1
(22) Date of filing: 03.07.2020
(51) Int. Cl.: A61K 9/52, A61K 31/197, A61K 31/198, A61K 35/64, A61K 36/48, A61K 36/484, A61K 36/537, A61K 47/00, A61P 15/12

(54) **TREATMENT FOR HOT FLUSHES, AND RE-ESTABLISHING A MENSTRUAL CYCLE DURING THE PERIMENOPAUSE**

(30) Priority: 30.07.2019 RU 2019124021
(71) Applicant: Obschestvo S Ogranichennoy Otvetstvennostju "Parapharm", Penza, 440026 (RU)
(72) Inventor: STRUKOV, Villoriy Ivanovich, Penza, 440018 (RU); PETROVA, Elena Vladimirovna, Penza, 440018 (RU); PROKHOROV, Mikhail Dmitrievich, Moscow, poseleniye Desenovskoe, 142793 (RU); KUZMINA, Elena Nikolaevna, selo Bessonovka, 442780 (RU); KURUS, Alexey Nikolaevich, Zarechniy, 442960 (RU); TRIFONOV, Vyaceslav Nikolaevich, Zarechniy, 442960 (RU); ELISTRATOVA, Julia Anatolievna, Penza, 440026 (RU); ELISTRATOVA, Polina Dmitrievna, Penza, 440026 (RU); ELSTRATOVA, Sofia Dmitrievna, Penza, 440026 (RU); ELISTRATOV, Georgiy Maksimovich, Penza, 440018 (RU); ELISTRATOV, Konstantin Gennadievich, Penza, 440061 (RU); KURUS, Natalia Vyacheslavovna, Penza, 440026 (RU); FEDOROV, Alexander Viktorovich, Kuznetsk, 442530 (RU); KRUTYAKOV, Evgeniy Nikolaevich, Penza, 440064 (RU); ANDREEVA, Elena Stanislavovna, Penza, 440028 (RU); ELISTRATOVA, Tatyana Viktorovna, Penza, 440018 (RU); KHOMYAKOVA, Irina Vladimirovna, Penza, 440068 (RU)
(74) Representative: Engel, Christoph Klaus
(86) International application number: PCT/RU2020/000327
(87) International publication number: WO 2021/020997

(57) **Abstract**

The invention relates to an agent and method for the treatment of hot flushes, and for re-establishing a menstrual cycle during the perimenopause. The claimed agent contains: β-alanine, HDBA organic complex, glycine, one or more medicinal plants containing phytoestrogens in an amount of at least 1 mg/g, and excipients. The method involves administering the claimed agent orally and/or sublingually in a dose from 500 mg to 1.5 g, 1-3 times a day for up to 3 years. The agent described above promotes normalisation of the menstrual cycle and relief from hot flushes, allows not only the level of estrogens but also the concentration of androgens to be safely normalised, and significantly alleviates even the most severe symptoms of the perimenopause, the hot flushes, by reducing the amount and the intensity thereof.

## Description

The Invention relates to medicine, especially to pharmaceutical industry, and is intended for restoring menstrual cycle and general welfare in women in perimenopause.

Climax is a natural condition of the female body associated with the change in hormonal status and a gradual decrease in reproductive function. Unfortunately, it is rarely well tolerated.

Climacteric syndrome is< first of all, a complex of neuropsychiatric, vegetative vascular and metabolic and trophic disorders.

The following symptoms are distinguished:
1. Early symptoms:
   - Vasomotor (hot flashes, hyperhidrosis, night sweats, drops in arterial pressure, palpitations, tachycardia, headaches, weakness, chills);
   - Emotional and mental (mood lability, cognitive disorders (loss of memory and concentration), anxiety, irritability, depression, dyssomnia, insomnia).
2. Intermediate symptoms:
   - urogenital (urogenital syndrome, vaginal dryness, genital tract atrophy, loss of libido, dyspareunia (pain during intercourse), itch, painful urination, cystourethritis, true incontinence of urine, genital prolapse);
   - Skin changes and its appendices (skin dryness, dry and brittle nails, wrinkles, dryness and loss of hair).
3. Late symptoms:
   - neuroendocrine (impaired glucose tolerance, visceral obesity);
   - cardiovascular (atherogenic dyslipidemia (decrease in HDLP, increase in LDLP and cholesterol in blood), arterial hypertension, homeostasis changes (increased levels of fibrinogen and blood coagulation factors), coronary heart disease, thrombosis/strokes).

Modern pharmaceutical industry provides two types of medicines for relieving climacteric symptoms: non-hormonal and hormonal. Both replenish estrogen deficiency in the female body. The former content substances similar to human estradiol (phytoestrogens), the latter - synthetic analogs of female sex hormones.

Why namely estrogens are included in every such medicine? The point is that the human brain has a specific section - hypothalamus that controls arterial pressure, vascular tone, the function of excretory glands, thermoregulation, emotions, and gastrointestinal motility. This section has a large numbers of sensitive nerve endings - receptors responsive to estrogens. As ovarian function fades with age, the levels of female sex hormones drop sharply, and the functioning of the hypothalamus is impaired. For a long time, this served as the explanation of unpleasant climacteric symptoms.

The medicines used in climax provide the restoration of estrogen blood levels in women up to normal and slightly improve the quality of life, however, unfortunately, they are not able to remove perimenopausal symptoms completely. The most severe manifestation of menopause, hot flashes, is often not corrected. The minus of the medicines containing synthetic analogs of female sex hormones is a large number of contraindications for using them and serious adverse effects.

So, hormone replacement therapy is strongly contraindicated for liver diseases, varices, thrombophlebitis, endometriosis, endometrial polyps, and several other pathologies found in most women over 40.

Numerous studies show that the consequences of using "non-endogenous" hormones can be malignant formations in the reproductive system organs, heart attacks, deep vein thrombosis, blockage of pulmonary artery and its branches with blood clots and several other diseases and conditions.

Obviously, the existing medicines roughly interfere physiological processes and do not take into account some important factor in the development of climacteric symptoms.

The condition of a woman in menopause depends not only on estrogens.

Recent studies have led researchers to suggest that the normal course of physiological processes during climax needs not only estrogens but also sufficient amounts of androgens. Receptors to male sex hormones have been found in the cells of almost all tissues of the woman's body - the mammary glands, uterus, ovaries, vagina, as well as the heart, blood vessels, lungs, brain and spinal cord, bladder, ureter, skin, lungs, gastrointestinal tract, bone marrow, synovial membrane, muscle, fatty and bone tissues.

The combined action of testosterone and estrogens has a more powerful effect on the woman's body than the effect of using them separately. For the normal course of physiological processes during climax, both estrogens and androgens are necessary. None of the medicines used for climax and being present in pharmaceutical market meets this requirement.

Unfortunately, the testosterone levels in woman's blood begins to drop after 20 years, and by the age of 40 is 50% of its original levels (Zumoff B., Strain G., Miller L. et al.). This cannot but affect the physical and mental state. Androgen deficiency cause not only sexual and reproductive disorders but also mood changes manifested in the form of increased anxiety, irritability, depression, decrease in bone mass (osteoporosis) and muscle mass (sarcopenia), obesity, cognitive disorders, urination, and other disorders.

Male sexual hormones, and namely their deficiency, are also responsible for the hot flashes in women. The fact is that it is the hypothalamus that plays the central role in the regulation of the body temperature (as well as in sweating control). This section of the brain contains the centers of thermoregulation that coordinate numerous complex processes, which ensure the maintenance of body temperature at a constant level. It has been experimentally established that the level of testosterone in the hypothalamus is 10 times higher than the level of estradiol. The experts are convinced that this is a reliable confirmation of the significant role of androgens in thermoregulation and the occurrence of hot flashes. The men who take medicines that counteract the effects of testosterone, as well as those of them who underwent surgery to remove the sexual glands, have also hot flashes as a result of sharp decline in testosterone levels. What is the mechanism of occurrence of hot flashes?

Presumably, the deficit of testosterone leads to an accumulation of low density lipoproteins ("bad" cholesterol), blood thickening, and the constriction of the blood vessels. It influences inevitably the arterial pressure - it increases, hot flashes, headaches occur, the risk of heart diseases and stroke increases.

Hormone replacement therapy using synthetic testosterone analogs helps to reduce hot flashes, vaginal dryness, improve mood, stop bone loss, increase muscle strength, reduce body weight and blood cholesterol levels, increase libido, and has a slight stimulating effect on the mammary gland tissue. However, women have to pay with their health for the removal of menopause symptoms, since synthetic androgens can provoke the development of atherosclerosis, invasive cancer of the mammary gland (it gives extensive metastases to other organs and tissues) and other serious diseases.

To normalize the women's condition in premenopausal and menopausal periods, non-hormonal medicines are also used. So, a medicine and a set for normalizing functional disorders occurring in premenopausal and menopausal periods is known from prior art (Patent RU 2220712). The suggested medicine contains L-glutamine acid or its pharmaceutically acceptable salts, glycine, acidic calcium succinate, acidic magnesium succinate, acidic zinc fumarate, tocopherol acetate, and ammonium succinate. The medicine makes it possible to stop the developments of neurologic and osteoporotic pathological disorders, sleep disorders, depression in premenopausal and menopausal periods, as well as to treat pathological amenorrhea, prevent neurological manifestations of premenstrual syndrome, increase energetic status of the body, and has also antioxidant and adaptogenic effects, reduces weather dependence, normalizes the state of the endocrine system, has a sugar-reducing effect and the effect of reducing the intensity of menopausal bleedings.

The disadvantage of this medicine is that it does not restore the menstrual cycle, has no effect on estrogen and androgen levels, and does not remove hot flashes.

Another medicine known from prior art - PhytoMixforWomen - is a product developed based on the data of scientific and epidemiologic researches, which is a safe non-hormonal naturopathic biologically active dietary supplement for reducing and relieving the symptoms of menopause. It includes amino acid β-alanine and phytoestrogens - specific substances contained in plants including hop cones, flax and saffron seeds, containing also excipients for preparing pharmaceutical composition (PhytoMixforWomen, Innovative product for relieving menopausal symptoms, HealthyNews 08/08/2016).

From the description of the supplements it follows that its main component is beta-alanine - an amino acid playing an important role in relieving such symptoms as hot flashes, night sweating, drops in arterial pressure, which menopausal women suffer often from. The intake of beta-alanine helps to reduce the occurrence and intensity of hot flashes, normalize the cycle of "sleep - wakefulness", improve psycho-emotional state.

This amino acid does not belong to essential acids, it is produced in the body by a natural way, however, its production gradually reduces with age, therefore beta-alanine should be taken with supplements.

The effectiveness of beta-alanine is explained by the fact that this amino acid is involved in several mechanism simultaneously responsible for thermoregulation of the body. Firstly, beta-alanine has a direct effect on the center of thermoregulation providing a quick effect (hot flashes are stopped in some minutes). Furthermore, it is part of carnosine helping to stabilize an energy metabolism and gradually normalize thermoregulation.

Beta-alanine is also required for the synthesis of pantothenic acid (vitamin B5). It, in turn, is necessary for the production of coenzyme A - an enzyme involved in many biochemical processes. Due to the accumulation of carnosine and pantothenic acid in the body, energy generation and regeneration processes are optimized, which has a beneficial effect on the function of thermoregulation.

Studies have shown that the intake of beta-alanine is an effective and safe alternative to hormone replacement therapy. Correcting the physiological and emotional state of women, it has almost of side effects. For instance, taking beta-alanine does not result in fluid retention in the body, as is often the case in HRT, when a woman gains weight due to the accumulation of water in the tissues. Beta-alanine intake does not cause addiction or drowsiness.

The disadvantages of this known medicine are as follows:
- When taking it, women experience the effects of tingling in hands and feet, redness of the face and neck.
- After a year of taking PhytoMixforWomen, an addictive effect is observed since this medicine does not help to normalize the hormonal status in women.
- It does not restore the menstrual cycle.

The closest prior art of the claimed composition is the composition and method for the treatment of androgen deficiency in women containing entomological proteins based on drone brood for reducing androgen deficiency in menopause (Patent RU 2577225). However, when using this composition, the following difficulties arose: in spite of the increase in androgen levels and the normalization of the menstrual cycle, the women continued to have the symptoms of hot flashes, which significantly reduced the quality of life and did not motivate them to take the composition. As a result, women "capitulated" to menopausal symptoms and wished only to end as soon as possible their sufferings - to end the period of hot flashes. I.e., women did not seek to maintain their menstruations (their reproductive cycle), which was the aim of the composition according to the Patent 2577225.

The objective that is suggested to solve by using the claimed invention is to develop a composition for treating hot flashes and restoring menstrual cycle during perimenopause in women and a method of using it.

To solve this objective, the composition for treating hot flashes and restoring menstrual cycle during perimenopause is claimed, comprising in % by mass:
B-alanine 0.1 - 60% by mass,
HDBA organic complex (drone brood homogenate adsorbed (lactose, glucose, fructose, drone brood) 5 - 40% by mass,
Glycine 9 - 40% by mass,
One or several medicinal plants 2.5 - 40% by mass, containing phytoestrogens not less than 1.0 mg/g.
The rest - excipients up to 100% by mass.

The following substances can be used as excipients: wheat starch, potato starch, corn starch, rice starch, pre-gelatinized starch, glucose, sucrose, fructose, lactose, basic magnesium carbonate, magnesium oxide, sodium chloride, sodium hydrocarbonate, gelatin, microcrystalline cellulose, powdered cellulose, methylcellulose, sodium salt of carboxymethylcellulose, calcium carbonate, calcium phosphate disubstituted, glycine, dextrin, amylopectin, ultraamylopectin, xylitol, sorbitol, mannitol, aspartame, dulcin, citric acid, cocoa, pectin, aerosil, talc, polyethylene oxide - 4000, stearic acid, calcium and magnesium stearate, sodium stearyl fumarate.

As medicinal plants containing phytoestrogens, red clover extract in powdered form, licorice root, salvia leaves can be used.

A method for treating hot flashes and restoring the menstrual cycle during perimenopause is also claimed, comprising the administration of the claimed composition orally and/or sublingually in the dose of 500 mg to 1.5 g 1-3 times per day for up to 3 years. The improvement of the patient's condition, as measured by a decrease in the severity of hot flashes, as a rule, occurs on the same day, and the normalization of the menstrual cycle occurs within 1-3 months from the start of taking the composition. Subsequently, depending on the patient's condition, the dose of the composition may be reduced to 500 mg per day. The duration of treatment depends on the woman's desire to prolong her menstruation. The authors of the invention have experience of monitoring women taking this composition for three years.

The lower limit of the dosage value is explained by the ineffectiveness of the claimed composition on the severity and frequency of hot flashes, and the upper limit is explained by the expediency of taking the composition and the greater likelihood of allergic reactions.

The technical results of the claimed invention are as follows:
- Normalization of the menstrual cycle and elimination of hot flashes. The authors belong to the therapeutic school that believes that the longer a woman maintains her menstrual cycle, the better it is for her health.
- Combined use of the said components has not been previously applied, the high efficiency of the treatment is due to the combined action of the components used.
- The composition provides a safe normalization of not only estrogen levels, but also androgen concentration, significantly reduce even severest manifestations of perimenopause - hot flashes, reducing their frequency and severity. All components of the composition are close to the human body and do not disturb the natural processes occurring in it.
- Absence of proliferative activity in the myometrium and endometrium, and in the mammary glands.
- Absence of the effects of tingling of hands and feet, redness of the face and neck in women taking the composition.
- Absence of the addictive effect for 3 years.

Essence of the invention. For preparing the pharmaceutical composition, β-alanine powder is provided. Drone brood can be provided in the form of adsorbed drone brood homogenate based on lactose, glucose, fructose or any combination thereof. Glycine is provided in the form of powder. As for the medicinal plant, any medicinal plant containing phytoestrogens can be selected, or a group of medicinal plants containing phytoestrogens. The medicinal plant can be prepared in the form of powder or dry extract containing phytoestrogens no less than 1 mg/g, for example, red clover extract, licorice root (ground vegetable raw materials, for example, in the form of powder), salvia leaves (ground vegetable raw materials, for example, in the form of powder), but not limited to them. The choice of specific medicinal plants and the ratio thereof depends on the availability of raw material base at the time of production. Excipients such as fillers, binders (for wet granulation), leavening and antifriction agents can also be used. Fillers can be, but not limited to: lactose, hydroxyl-propylmethylcellulose, microcrystalline cellulose. Humidifiers can be, but limited to: purified water, ethanol, starch paste, sugar syrup, solutions: carboxymethyl-cellulose (CMC), Na CMC, oxypropylmethyl-cellulose (OPMC), oxyethylcellulose (OEC), pre-gelatinized starch, polyvinyl alcohol, polyvinyl-pyrrolidone (PVP), alginic acid, sodium alginate, gelatin.

These powders are mixed in a standard mixer for 15 minutes until homogeneous.

From the finished mixture, gelatin capsules are made in the dose of 500 mg to 1.5 g, or the finished mixture is tableted, in which a humidifier is added to the finished mixture, then the mixture is granulated and dried in a granulator. After drying, the granulation process is carried out again, ducting with sliding and lubricating substances: starch, calcium stearate, the resulting mass is tableted in tablet presses. The weight of one tablet is 505 mg.

The invention is explained by Table 1 - Comparative study of the condition of women according to the modified Kupperman Modified Menopausal Index (KMMI) as modified by E.V.Uvarova (1982) at the beginning of taking the composition and 60 days after the start of taking the composition, where the diagrams for Composition 1 - Kupperman index values for the composition described in the prototype, the diagrams for Composition 2 - Kupperman index values for PhytoMixforWomen, the diagrams for Composition 3 - Kupperman index values for the claimed composition.

### Example 1. Obtaining the composition in tableted form.

For 1000 kg of the composition according to the invention, are provided:
Powdered β-alanine in the amount of 100 kg,
Powdered glycine in the amount of 350 kg,
Drone brood homogenate adsorbed based on lactose in the amount of 350 kg,
Powdered red clover extract, the content of phytoestrogens in terms of isoflavones 2.0 mg/g - in the amount of 50 kg.
Lactose as filler is added in the amount of 132.5 kg.

The powders are mixed in a mixer for 15 minutes, then humidified with 3% hydroxypropylmethylcellulose solution. The amount of humidifier does not exceed 7.5 kg diluted in 242.5 kg of water. This is followed by the processes of granulation and drying in a granulator. As a result, water evaporates. After drying, the process of granulation is carried out again, ducting with starch and calcium stearate in the amount of 10 kg in a mixer, tableting of the tablet mass in the amount of 1000 kg in tablet presses. The weight of one tablet is 505 mg. Depending on the available equipment, the production process can be changed according to the requirement of the pharmacopoeia.

### Example 2. Obtaining the composition in tableted form.

For 1000 kg of the composition according to the invention, are provided:
Powdered β-alanine in the amount of 400 kg,
Powdered glycine in the amount of 100 kg,
Drone brood homogenate adsorbed based on lactose in the amount of 200 kg,
Powdered licorice root (ground vegetable raw material), the content of phytoestrogens 140 mg/g - in the amount of 250 kg.
Lactose as filler is added in the amount of 32.5 kg.

The powders are mixed in a mixer for 15 minutes, then humidified with 3% hydroxypropylmethylcellulose solution. The amount of humidifier does not exceed 7.5 kg diluted in 242.5 kg of water. This is followed by the processes of granulation and drying in a granulator. As a result, water evaporates. After drying, the process of granulation is carried out again, ducting with sliding agents - starch and calcium stearate in the amount of 10 kg in a mixer, tableting of the tablet mass in the amount of 1000 kg in tablet presses. The weight of one tablet is 505 mg. Depending on the available equipment, the production process can be changed according to the requirement of the pharmacopoeia.

### Example 3. Obtaining the composition in tableted form.

For 1000 kg of the composition according to the invention, are provided:
Powdered β-alanine in the amount of 600 kg,
Powdered glycine in the amount of 200 kg,
Drone brood homogenate adsorbed based on lactose in the amount of 50 kg,
Powdered salvia leaves (ground vegetable raw material), the content of phytoestrogens 4.0 mg/g - in the amount of 25 kg.
Lactose as filler is added in the amount of 107.5 kg.

The powders are mixed in a mixer for 15 minutes, then humidified with 3% hydroxypropylmethylcellulose solution. The amount of humidifier does not exceed 7.5 kg diluted in 242.5 kg of water. This is followed by the processes of granulation and drying in a granulator. As a result, water evaporates. After drying, the process of granulation is carried out again, ducting with sliding agents - starch and calcium stearate in the amount of 10 kg in a mixer, tableting of the tablet mass in the amount of 1000 kg in tablet presses. The weight of one tablet is 505 mg. Depending on the available equipment, the production process can be changed according to the requirement of the pharmacopoeia.

### Example 4. Obtaining the composition in capsulated form.

The process of obtaining the mixture for encapsulation per 1000 kg is as follows:
Powdered β-alanine in the amount of 150 kg,
Powdered glycine in the amount of 400 kg,
Drone brood homogenate adsorbed based on lactose in the amount of 350 kg,
Powdered licorice root with the content of phytoestrogens 70 mg/g - in the amount of 50 kg.
Excipients - starch, aerosil, calcium stearate in the amount of 50 kg are added to give the product free-flowing and anti-caking properties.

This is followed by a mixing process in a mixer for 15 minutes, then by an encapsulation process with a capsule filling machine. Hard gelatin capsules are used. Depending on the available equipment, the production process can be changed according to the requirement of the pharmacopoeia.

### Example 5. Obtaining the composition in capsulated form.

The process of obtaining the mixture for encapsulation per 1000 kg is as follows:
Powdered β-alanine in the amount of 550 kg,
Powdered glycine in the amount of 100 kg,
Drone brood homogenate adsorbed based on lactose in the amount of 100 kg,
Slavia leaves (ground vegetable raw material), the content of phytoestrogens 1.2 mg/g - in the amount of 200 kg.
Excipients - starch, aerosil, calcium stearate in the amount of 50 kg are added to give the product free-flowing and anti-caking properties.

This is followed by a mixing process in a mixer for 15 minutes, then by an encapsulation process with a capsule filling machine. Hard gelatin capsules are used. Depending on the available equipment, the production process can be changed according to the requirement of the pharmacopoeia.

### Example 6. Obtaining the composition in capsulated form.

The process of obtaining the mixture for encapsulation per 1000 kg is as follows:
Powdered β-alanine in the amount of 200 kg,
Powdered glycine in the amount of 200 kg,
Drone brood homogenate adsorbed based on lactose in the amount of 200 kg,
Powdered red clover extract, the content of phytoestrogens 7.4 mg/g - in the amount of 350 kg.
Excipients - starch, aerosil, calcium stearate in the amount of 50 kg are added to give the product free-flowing and anti-caking properties.

This is followed by a mixing process in a mixer for 15 minutes, then by an encapsulation process with a capsule filling machine. Hard gelatin capsules are used. Depending on the available equipment, the production process can be changed according to the requirement of the pharmacopoeia.

### Example 7. Obtaining the composition in capsulated form.

The process of obtaining the mixture for encapsulation per 1000 kg is as follows:
Powdered β-alanine - 200 kg,
Powdered glycine - 200 kg,
Drone brood homogenate adsorbed based on lactose - 200 kg,
Powdered red clover extract, salvia leaves (ground vegetable material) in the ratio of 1:1 in the amount of 350 kg.
Excipients - starch, aerosil, calcium stearate in the amount of 50 kg are added to give the product free-flowing and anti-caking properties.

This is followed by a mixing process in a mixer for 15 minutes, then by an encapsulation process with a capsule filling machine. Hard gelatin capsules are used. Depending on the available equipment, the production process can be changed according to the requirement of the pharmacopoeia.

### Example 8. Obtaining the composition in capsulated form.

The process of obtaining the mixture for encapsulation per 1000 kg is as follows:
Powdered β-alanine in the amount of 550 kg,
Powdered glycine in the amount of 100 kg,
Drone brood homogenate adsorbed based on lactose in the amount of 100 kg,
Salvia leaves (ground vegetable material), powdered red clover extract, powdered licorice root in the ratio of 1:1:1 in the amount of 200 kg.
Excipients - starch, aerosil, calcium stearate in the amount of 50 kg are added to give the product free-flowing and anti-caking properties.

This is followed by a mixing process in a mixer for 15 minutes, then by an encapsulation process with a capsule filling machine. Hard gelatin capsules are used.

### Example 9. Obtaining the composition in tableted form.

The process of obtaining the mixture for encapsulation per 1000 kg is as follows:
Powdered β-alanine in the amount of 600 kg,
Powdered glycine in the amount of 200 kg,
Drone brood homogenate adsorbed based on lactose in the amount of 50 kg,
Powdered red clover extract, salvia leaves (ground vegetable material) in the ratio of 1:1 in the amount of 25 kg.
Lactose as filler is added in the amount of 107.5 kg.

The powders are mixed in a mixer for 15 minutes, then humidified with 3% hydroxypropylmethylcellulose solution. The amount of humidifier does not exceed 7.5 kg diluted in 242.5 kg of water. This is followed by the processes of granulation and drying in a granulator. As a result, water evaporates. After drying, the process of granulation is carried out again, ducting with sliding agents - starch and calcium stearate in the amount of 10 kg in a mixer, tableting of the tablet mass in the amount of 1000 kg in tablet presses. The weight of one tablet is 505 mg.

### Example 10. Obtaining the composition in tableted form.

The process of obtaining the mixture for encapsulation per 1000 kg is as follows:
Powdered β-alanine in the amount of 400 kg,
Powdered glycine in the amount of 100 kg,
Drone brood homogenate adsorbed based on lactose in the amount of 200 kg,
Salvia leaves (ground vegetable material), powdered red clover extract, powdered licorice root in the ratio of 1:1:1 in the amount of 250 kg.
Lactose as filler is added in the amount of 32.5 kg.

The powders are mixed in a mixer for 15 minutes, then humidified with 3% hydroxypropylmethylcellulose solution. The amount of humidifier does not exceed 7.5 kg diluted in 242.5 kg of water. This is followed by the processes of granulation and drying in a granulator. As a result, water evaporates. After drying, the process of granulation is carried out again, ducting with sliding agents - starch and calcium stearate in the amount of 10 kg in a mixer, tableting of the tablet mass in the amount of 1000 kg in tablet presses. The weight of one tablet is 505 mg.

### Example 11. The effectiveness of the composition has been tested on women volunteers.

Study inclusion criteria were as follows:
- age of 40-60 years,
- presence of vasomotor, psycho-emotional and sexual disorders due to climacteric syndrome,
- changes in the menstrual cycle at the age of 40 and over not associated with diseases, which is confirmed by a gynecologist (amenorrhea or irregular menstruation during pre-, peri- and postmenopause).

Study exclusion criteria were as follows:
- a history of allergic reactions, including hypersensitivity to the components of "Femo-Clim" (the claimed composition);
- HRT with sex steroid the use of psychotropic drugs;
- addiction to psychoactive substances;
- conditions that threaten the patient's life.

30 women volunteers in perimenopause were divided into 6 groups:
Group 1 - 5 women who received the claimed composition in the ratio of components according to Example 1 in the form of a tablet weighing 500 mg. Patients received the claimed composition sublingually 2 times a day.
Group 2 - 5 women who received the claimed composition in the ratio of components according to Example 3 in the form of a tablet weighing 500 mg. Patients received the claimed composition sublingually 3 times a day.
Group 3 - 5 women who received the claimed composition in the ratio of components according to Example 4 in the form of a tablet weighing 500 mg. Patients received the claimed composition sublingually 2 times a day.
Group 4 - 5 women who received the claimed composition in the ratio of components according to Example 5 in the form of a tablet weighing 500 mg. Patients received the claimed composition sublingually 3 times a day.
Group 5 - 5 women who received the claimed composition in the ratio of components according to Example 9 in the form of a tablet weighing 500 mg. Patients received the claimed composition sublingually 3 times a day.
Group 6 - 5 women who received the claimed composition in the ratio of components according to Example 8 in the form of a tablet weighing 500 mg. Patients received the claimed composition sublingually 2 times a day.

During treatment, relief of the symptoms of hot flashes was observed, which occurred already in the first days of treatment, and the menstrual cycle was restored during the first month of treatment.

For three years, the women of all 3 groups managed to maintain menstruation and achieve a significant reduction in the frequency and severity of hot flashes, practically reducing them to zero. The women participating in the study were regularly examined by a gynecologist and mammologist, they did not have any pathological changes in the tissues of the endometrium and mammary gland, there was no increase also in blood thrombogenic potential.

The study of the hormone levels in the blood of tested patients has shown that the claimed composition increases the levels of estradiol and testosterone in the blood, which indicates a decrease in the biological age of patients.

So, the average values of hormone levels in the blood before the study were as follows:
Estradiol - 75.6 ± 0.4 pmol/l,
Total testosterone - 1.7 ± 0.3 nmol/l.

180 days after taking the claimed composition, the hormone levels were as follows:
Estradiol - 82.3 ± 0.6 pmol/l,
Total testosterone - 2.3 ± 0.2 nmol/l.

The women participating in the study were pharmacists of one of the Penza pharmacy chains, who, prior to the start of the study, had used all medicines for menopause available in the pharmacy chain. However, the effects of those medicines were short-lived: 2 to 6 months, the effect of hot flashes was not completely eliminated, addiction to the medicines developed, which manifested itself in an increase in hot flashes (frequency and severity).

The medicines used by these women (before this study) had no effect on the normalization of the menstrual cycle.

The claimed composition did not only restore the normal menstrual cycle, but also influenced significantly the frequency and severity of hot flashes, practically eliminating them.

None of the women participating in the study did not agree to discontinue the claimed composition since they have seen no real alternative among the medicines available on the pharmaceutical market.

The authors have no experience of using the claimed composition for more than 3 years. The research continues.

Example 12. Comparative study of the condition of women according to the modified Kupperman Modified Menopausal Index (KMMI) as modified by E.V.Uvarova (1982) has shown the following. The study involved 3 groups of women, 15 patients each.

Group 1 received the composition described in the prototype in the dose of 300 mg daily.

Group 2 received PhytoMixforWomen according to the instructions.

Group 3 received the claimed composition 1 tablet in the morning and in the evening according to Example 2.

The result of the comparative study before treatment and after 60 days of treatment is shown in Table 1. The results obtained indicate a significant decrease in Kupperman index during the treatment in women of Group 3. This indicates a high effectiveness of the claimed composition in reducing the severity of climacteric syndrome.

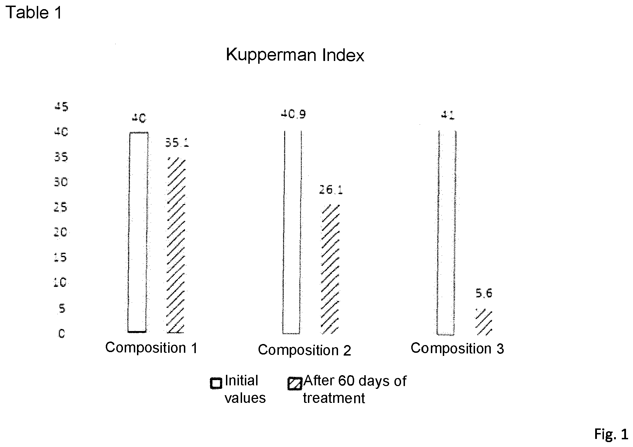

## Claims

1. A composition for treating hot flashes and restoring the menstrual cycle in the period of perimenopause, comprising % by mass:
β-alanine 0.1-60% by mass,
HDBA organic complex 5-40% by mass,
Glycine 9-40% by mass,
one or more medicinal plants 2.5-40% by mass, containing phytoestrogens in the amount of not less than 1 mg/g,
the rest - excipients up to 1005 by mass.

2. The composition according to Claim 1 **characterized in that** wheat starch, potato starch, corn starch, rice starch, pre-gelatinized starch, glucose, sucrose, fructose, lactose, basic magnesium carbonate, magnesium oxide, sodium chloride, sodium hydrocarbonate, gelatin, microcrystalline cellulose, powdered cellulose, methylcellulose, sodium salt of carboxymethylcellulose, calcium carbonate, calcium phosphate disubstituted, glycine, dextrin, amylopectin, ultraamylopectin, xylitol, sorbitol, mannitol, aspartame, dulcin, citric acid, cocoa, pectin, aerosil, talc, polyethylene oxide - 4000, stearic acid, calcium and magnesium stearate, sodium stearyl fumarate can be used as excipients.

3. The composition according to Claim 1 **characterized in that** red clover extract in the form of powder, licorice root, salvia leaves can be used as medicinal plant/plants containing phytoestrogens.

4. A method for treating hot flashes and restoring the menstrual cycle in the period of perimenopause, comprising the administration of the composition according to Claim 1 orally and/or sublingually in the dose of 500 mg to 1.5 g 1-3 times per day for up to 3 years.

5. The method according to Claim 4 **characterized in that** as the patient's condition improves, the severity of hot flashes decreases, and the menstrual cycle normalizes, the dose of the composition taken may be reduced to 500 mg 1 time per day.
